(19) Europäisches Patentamt
European Patent Office
Office européen des brevets

(11) Veröffentlichungsnummer: **0 427 040 A1**

(12) # EUROPÄISCHE PATENTANMELDUNG

(21) Anmeldenummer: **90120276.2**

(22) Anmeldetag: **23.10.90**

(51) Int. Cl.5: **D21H 21/30**, C07C 43/225, C07C 25/24, C07C 15/58, C07C 317/22, C07C 317/14, C07C 323/09

(30) Priorität: **04.11.89 DE 3936793**

(43) Veröffentlichungstag der Anmeldung: **15.05.91 Patentblatt 91/20**

(84) Benannte Vertragsstaaten: **AT CH DE FR GB IT LI SE**

(71) Anmelder: **BAYER AG**
**W-5090 Leverkusen 1 Bayerwerk(DE)**

(72) Erfinder: **Seng, Florin, Dr.**
**Am Katterbach 46**
**W-5060 Bergisch Gladbach 2(DE)**
Erfinder: **Kysela, Ernst, Dr.**
**Virchowstrasse 14**
**W-5060 Bergisch Gladbach 1(DE)**
Erfinder: **König, Joachim, Dr.**
**Auf dem Broich 25**
**W-5068 Odenthal(DE)**
Erfinder: **Schuffenhauer, Erhard, Dipl.-Ing.**
**Friedrich-Bayer-Strasse 13**
**W-5090 Leverkusen 1(DE)**
Erfinder: **Bieber, Werner**
**Gladbacher Weg 13**
**W-5653 Leichlingen 1(DE)**
Erfinder: **Hunke, Bernhardt**
**Am Burghart 15**
**W-5202 Hennef/Sieg(DE)**

(54) **Weisstönerhaltige Papierstreichmassen.**

(57) Papierstreichmassen enthaltend Verbindungen der Formel

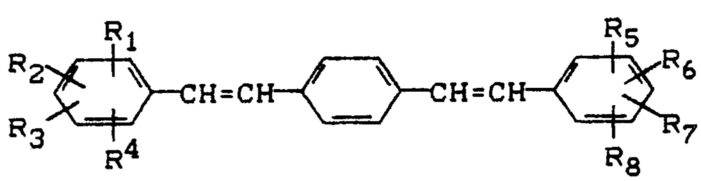

(I)

worin $R_1$, $R_2$, $R_3$ und $R_5$, $R_6$, $R_7$ H, F, Cl oder $CH_3$ und $R_4$ und $R_8$ H, O-$C_1$-$C_4$-Alkyl, S-$C_1$-$C_4$-Alkyl, $SO_2C_1$-$C_4$-Alkyl, $CF_3$, $OCF_3$ oder $SCF_3$ bedeuten, sind kostengünstig und zeichnen sich durch einen hohen Weißgrad aus.

EP 0 427 040 A1

## WEISSTÖNERHALTIGE PAPIERSTREICHMASSEN

Zur optischen Aufhellung von Streichmassen auf der Basis von Kunststoff-Dispersionen werden normalerweise substantive anionische Weißtöner eingesetzt. Diese Weißtöner zeigen jedoch in den Streichmassen nur sehr unbefriedigende Aufhell-Effekte, eine sehr niedrige Vergrauungsgrenze sowie eine geringe Lichtechtheit.

Es ist allgemein bekannt, die genannten Schwierigkeiten teilweise zu lösen, dadurch, das man hydrophile Cobinder oder Carrier zur Streichmasse zusetzt. (Vgl. "Das Papier" 36 (1982), 66, DE-A-3 502 038 und EP-A-43 790).

Auf diese Weise kann zwar der Weißeffekt deutlich verbessert werden, jedoch wird gleichzeitig die Wasser-Empfindlichkeit der gestrichenen Papiere erhöht.

Es ist weiterhin bekannt (vgl. DE-A 3 112 435), wasserlösliche Weißtöner auf bestimmte Kunststoffe, wie z.B. Harnstoff-oder Methylol-Harze, aufziehen zu lassen und diese aufgehellten Kunststoffe als Dispersion der Papierstreichmasse zuzusetzen.

Schließlich ist vorgeschlagen worden (DE-A 3 643 215), wasserunlösliche Salze von anionischen Weißtönern und langkettigen Fettaminen für den Papierstrich einzusetzen. Diese Vorschläge haben sich jedoch bisher wegen ihrer zu hohen Kosten oder wegen rheologischer Schwierigkeiten nicht durchsetzen können.

Es wurde nun überraschenderweise gefunden, daß man Streichmassen für den Papierstrich ohne die genannten Nachteile aufhellen kann, wenn man den Streichmassen Weißtöner der Formel

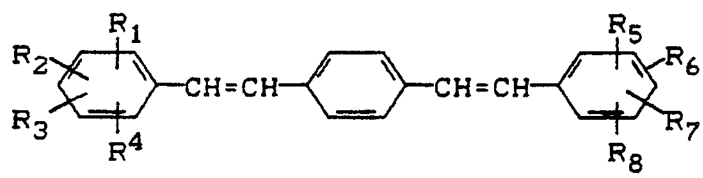

(I)

worin - unabhängig voneinander - $R_1$, $R_2$, $R_3$ und $R_5$, $R_6$, $R_7$ H, F, Cl oder $CH_3$ und $R_4$ und $R_8$ H, O-$C_1$-$C_4$-Alkyl, S-$C_1$-$C_4$-Alkyl, $SO_2C_1$-$C_4$-Alkyl, $CF_3$, $OCF_3$ oder $SCF_3$ bedeuten,
wobei zwei benachbarte Reste auch für $-OCH_2O-$ oder -CH=CH-CH=CH- stehen können,
zusetzt.

Vorzugsweise stehen die Alkylreste für Methylreste.

Gegenstand der Erfindung sind weiterhin neue Verbindungen der Formel I, worin
$R_4$ und $R_8$ F, $CF_3$, $OCF_3$ oder $SCF_3$ bedeuten und die übrigen Reste die oben genannte Bedeutung haben, mit der Maßgabe, das diese Reste nicht für Wasserstoff stehen, wenn $R_4$/$R_8$ F oder $CF_3$ bedeuten.

Die Distyryl-Verbindungen der Formel (I) können nach an sich bekannten Methoden dadurch hergestellt werden, das man eine Verbindung der Formel

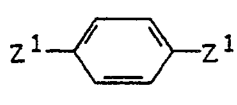

(II)

im gewünschten Verhältnis mit je einer Verbindung der Formeln

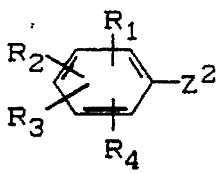

(IIIa) bzw.

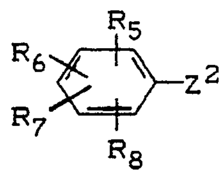

(IIIb)

umsetzt, wobei je eines der Symbole $Z_1$, bzw. $Z_2$ eine Formylgruppe und das andere eine Gruppierung der Formel

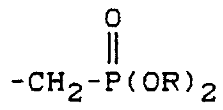

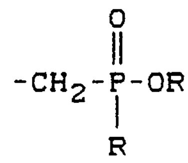

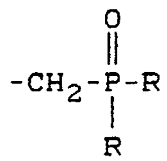

oder

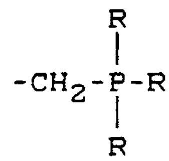

darstellt, worin
R einen $C_1$-$C_4$-Alkyl, einen $C_5$-$C_6$-Cycloalkyl- oder einen gegebenenfalls weitersubstituierten Arylrest, vorzugsweise einen Phenylrest bedeutet.

Auf diese Weise können symmetrische und unsymmetrische Verbindungen und Gemische aus symmetrischen und unsymmetrischen Verbindungen hergestellt werden.

Die hierbei als Ausgangsstoffe benötigten Phosphorverbindungen der Formeln (IIIa), (IIIb) und (II) werden erhalten, indem man Halogenmethylverbindungen, vorzugsweise Chlormethyl- oder Brommethylverbindungen der Formeln

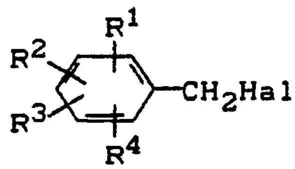

bzw.

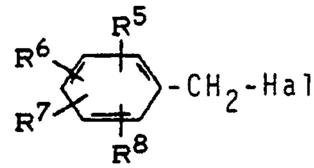

und

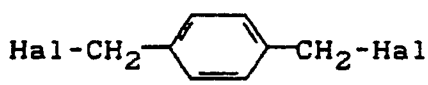

mit Phosphorverbindungen $P(OR)_3$, $R$-$P(OR)_2$, $RO$-$P(R)_2$ oder $P(R)_3$ umsetzt, wobei R die angegebene Bedeutung hat. Vorzugsweise bedeutet R an Sauerstoff gebunden $C_1$-$C_4$-Alkyl, an Phosphor gebunden dagegen Phenyl.

Zur Herstellung der Endstoffe kondensiert man die entsprechenden Komponenten in Gegenwart basischer Kondensationsmittel in organischen Lösungsmitteln.

Als Lösungsmittel wählt man vorteilhaft indifferente Lösungsmittel, beispielsweise Kohlenwasserstoffe wie Toluol oder Xylol oder Alkohole wie Methanol, Ethanol, Isopropanol, Butanol, Glykol, Glykolether wie 2-Methoxyethanol, Hexanol, Cyclohexanol, Cyclooctanol, ferner Ether wie Diisopropylether, Dioxan, Tetrahydrofuran; weiterhin Formamide, N-Methylpyrrolidon, Dimethylsulfoxid und Phosphorsäureamid. Bevorzugt sind Dimethylformamid, Dimethylacetamid und Phosphorsäure-trisdialkylamide, wobei Alkyl insbesondere $C_1$-$C_4$-Alkyl ist.

Als Kondensationsmittel kommen stark basische Verbindungen in Betracht wie Alkali- oder Erdalkalimetallhydroxide, Alkali- oder Erdalkaliamide und Alkali- oder Erdalkalimetallalkoholate, beispielsweise Kaliumhydroxid, Natriumhydroxid, Kalium-tert.-butylat, Natriumamid oder Natriummethylat, ferner die Alkaliverbindungen des Dimethylsulfoxids und Alkalihydride sowie gegebenenfalls Alkalimetalldispersionen.

Man arbeitet bevorzugt im Temperaturbereich von 0 bis 120° C.

Die erfindungsgemäßen Verbindungen (I) werden ebenfalls erhalten, wenn man die entsprechenden Aldehydanile in einem dipolar aprotischen Lösungsmittel wie Dimethylformamid in Gegenwart von basischen Kondensationsmitteln mit den entsprechenden Methylverbindungen umsetzt.

Die Verbindungen der Formel (I) zeigen im gelösten oder fein verteilten Zustand eine sehr starke blaue Fluoreszenz. Sie eignen sich einzeln oder als Mischung zum Weißtönen der verschiedensten synthetischen, halbsynthetischen oder natürlichen organischen Materialien.

Besonders bevorzugtes Anwendungsgebiet für die Verbindungen und Mischungen der Formel (I) ist der Einsatz in Papierstreichmassen.

Die Aufheller können direkt oder nach vorheriger Feinmahlung oder Flüssigformierung als Aufhellungsmittel eingesetzt werden.

Die Flüssigformierungen können wie folgt hergestellt werden:

Die Preßkuchen und/oder Pulver werden nach Zugabe eines oberflächenaktiven Stoffes und/oder Dispergiermittels und gegebenenfalls Wasser unter starkem Rühren homogenisiert. Danach kann eine Erhöhung der Menge dieser Hilfsmittel, gegebenenfalls auf die gesamte, für die Stabilität der Dispersion benötigte Menge, erfolgen.

Die erhaltene Suspension wird dann vorzerkleinert und naßgemahlen.

Die Vorzerkleinerung kann über Stein- oder Zahnkolloidmühlen erfolgen.

Die anschließende Naßzerkleinerung kann in Kolloid-, Schwing-, Kegel- und Vibromühlen sowie in Dissolvern oder in Kugelmühlen bzw. in Sub-Mikron-Dispersern erfolgen. Vorzugsweise werden jedoch kontinuierlich arbeitende Rührwerksmühlen mit Mahlkörpern, vorzugsweise solche aus $SiO_2$ von 0,1 bis 5 mm, insbesondere 0,2-2 mm, Durchmesser verwendet. Der Mahlvorgang ist beendet, wenn eine Korngröße des Produkts von < 5 $\mu$m, vorzugsweise < 0,5 $\mu$m erreicht ist.

Nach der Mahlbehandlung können gegebenenfalls noch weitere Mengen oberflächenaktiver Stoffe oder auch hydrotrope Substanzen wie z.B. Ethylenglykol oder Glycerin, Konservierungsmittel, Netzmittel, Entschäumer, Entstaubungsmittel und/oder Wasser zugegeben werden, soweit dies nicht schon zu einem früheren Zeitpunkt, z.B. vor der Mahlung, erfolgte.

Die nach diesem Verfahren hergestellten Dispersionen enthalten 5 bis 50 %, vorzugsweise 10 bis 30 % der erfindungsgemäßen, in Wasser schwer bzw. unlöslichen Aufhellersalze, 1 bis 50 %, vorzugsweise 5 bis 20 % oberflächenaktive Stoffe bzw. Dispergiermittel, 0 bis 5 % Konservierungsmittel und Wasser, wobei ein Teil des Wassers durch hydrotrope Substanzen ersetzt werden kann (% = Gewichtsprozent).

Als oberflächenaktive Stoffe kommen alle üblichen, kationischen und nichtionogenen oberflächenaktiven Stoffe in Betracht, wie sie beispielsweise in der DE-OS 2 334 769, Seiten 8 bis 10 (entspricht GB-PS 1 417 071) beschrieben sind.

Bevorzugt werden nichtionogene Tenside eingesetzt. Geeignete anionische Dispergiermittel sind übliche Formaldehydkondensationsprodukte von Arylsulfonamiden, sulfierten Diarylethern und Diarylsulfonen u.a.m.

Die Papierstreichmassen, in welche die erfindungsgemäß zu verwendenden Aufheller eingearbeitet werden, sind folgendermaßen aufgebaut: Kunststoff-Dispersionen auf Basis von Styrol-Butadien-, carboxylierten Styrol-Butadien- oder Polyvinylacetat-Copolymeren oder von Acrylsäureestern in Kombination mit Weißpigmenten, sowohl ohne als auch mit geringen Anteilen an hydrophilen Cobindern.

Die Einsatzmenge der Verbindungen I richtet sich nach dem angestrebten Aufhelleffekt. Im allgemeinen genügt 0,01 bis 0,5 Gew.-% reiner Wirksubstanz (bezogen auf den Feststoff der aufzuhellenden Papierstreichmasse). Ein besonderer Vorteil ist, daß je nach Streichmassen-Zusammensetzung die erreichbare Vergrauungsgrenze (bis zu welche ein weiterer Aufheller-Zusatz noch zusätzliche Effekte bringt) außerordentlich hoch liegt.

Beispiel 1

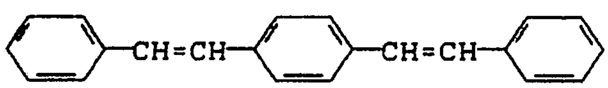

a) 40 g (0,1 Mol) p-Xylylendiethylbisphosphonat (93.4%ig) werden, zusammen mit 23.3 g (0.22 Mol) Benzaldehyd 100 ml Dimethylformamid vorgelegt. Dazu tropft man bei Raumtemperatur unter Stickstoff 39.6 g (0,22 Mol) einer 30%igen Natriummethylatlösung. Man rührt 2 Stunden bei Raumtemperatur und 30 min. bei 60 °C nach. Nach dem Abkühlen wird abgesaugt. Man erhält 19.8 g (70,2%) Distyrylbenzol in Form schwach gelber Kristalle die nach dem Umlösen aus Toluol/Tonsil bei 173 °C schmelzen. Extinktion in DMF: $\lambda_{max}$ = 355,9 nm; $\epsilon$ = 6600.

b) 250 g des kristallinen Aufhellers werden mit 200 g Benzyl-phenyl-phenol mit 14 Ethylenoxid und 550 g Wasser vermischt und auf einer Korundscheibenmühle einer Vorzerkleinerung unterzogen und solange gemahlen bis eine durchschnittliche Teilchengröße von kleiner 50 $\mu$m erhalten wird. Dann wird mit

Glasperlen von 0,2 mm Durchmesser in einer Rührwerksmühle auf eine durchschnittliche Teilchengröße von unter 0,5 µm gemahlen. Die erhaltene feine Dispersion kann sofort in die anwendungsgemäße Papierstreichmasse einearbeitet werden.

c) 300 g des kristallinen Aufhellers werden mit 150 g eines Formaldehyd-Kondensationsproduktes aus Ditolylethersulfonsäure-Na-Salz und 550 g Wasser versetzt und wie in Beispiel 1a behandelt. Nach der Feinmahlung werden noch 200 g Kondensationsprodukt aus Formaldehyd und Ditolylethersulfonsäure-Na-Salz sowie 200 g Kondensationsprodukt aus Formaldehyd und Naphthalinsulfonsäure-Na-Salz zugesetzt, sowie 50 g einer Mineralölemulsion (10 % wäßrig).

Anschließend wird die Suspension einem Sprühtrocknungsprozeß unterzogen und bei ca. 5 % Restfeuchte zu einem 30 % Wirksubstanz enthaltenden Granulat getrocknet, welches sich unter einem Schnellrührer in die anwendungsgemäße Papierstreichmasse einarbeiten läßt.

Beispiel 2

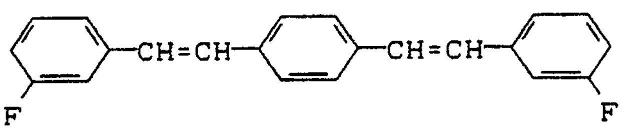

24,6 g (0,1 Mol) Fluorbenzylethylphosphonat und 6.5 g (0,05 Mol) Terephthalaldehyd werden unter Stickstoff in 50 ml Dimethylformamid vorgelegt. Bei Raumtemperatur tropft man 18 g (0,1 Mol) 30%ige Natriummethylatlösung zu und rührt 2 Stunden bei Raumtemperatur und 1 Stunde bei 60 °C nach. Nach dem Abkühlen wird abgesaugt und man erhält 24,5 g (77%) Bis-(m-fluorstyryl)-benzol als farblose Kristalle, die nach dem Umlosen aus Toluol/Tonsil bei 188-190 °C schmelzen. Extinktion in DMF: $\lambda_{max}$ = 355,9 nm; $\epsilon$ = 61 200.

Beispiele 3 - 48

In Analogie zu den Verfahren der Beispiele 1 und 2 werden die in nachfolgender Tabelle aufgeführten Weißtöner erhalten:

Tabelle

| Beispiel | Formel | Extinktion in DMF |
|---|---|---|
| 3 | 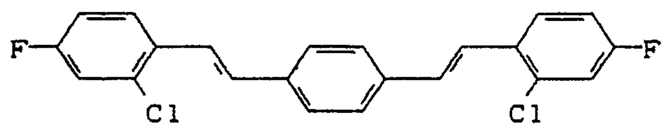 | λ = 359,9 nm<br>ε = 48 500 |
| 4 | 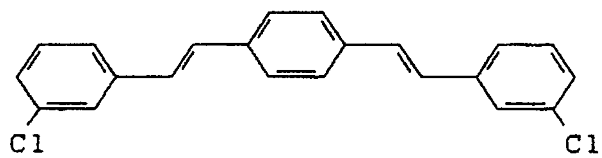 | λ = 358,4 nm<br>ε = 66 500 |
| 5 | 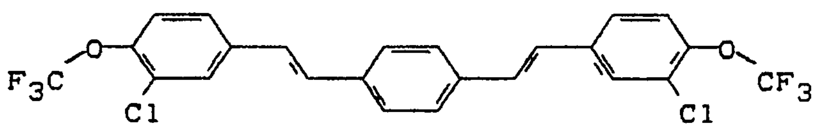 | λ = 359 nm<br>ε = 67 000 |

Tabelle (Fortsetzung)

| Beispiel | Formel | Extinktion in DMF |
|---|---|---|
| 6 | 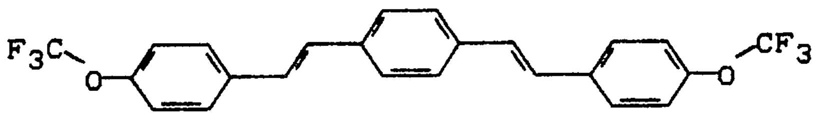 | λ = 357,1 nm<br>ε = 66 000 |
| 7 | 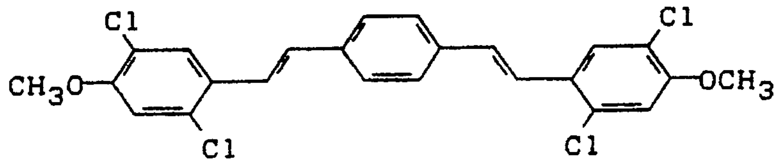 | λ = 371 nm<br>ε = 67 700 |
| 8 | 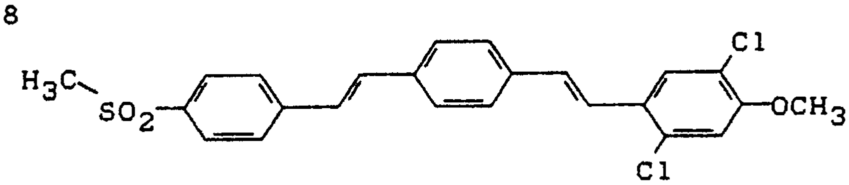 | λ = 370,4 nm<br>ε = 68 200 |
| 9 | 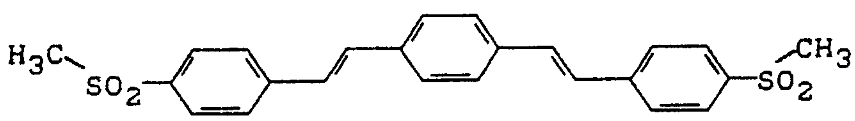 | λ = 369,7 nm<br>ε = 80 800 |
| 10 | 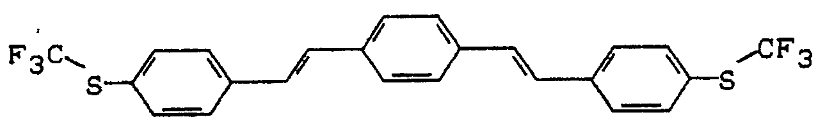 | λ = 368,5 nm<br>ε = 74 600 |
| 11 | 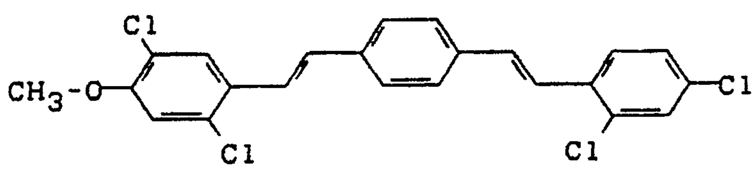 | λ = 371 nm<br>ε = 61 800 |

Tabelle (Fortsetzung)

| Beispiel | Formel | Extinktion in DMF |
|---|---|---|
| 12 | 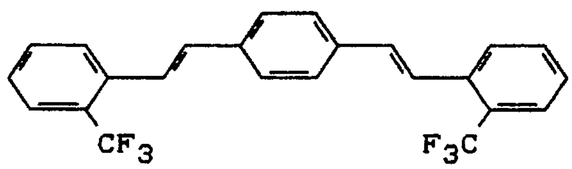 | $\lambda$ = 348,6 nm<br>$\varepsilon$ = 56 000 |
| 13 | 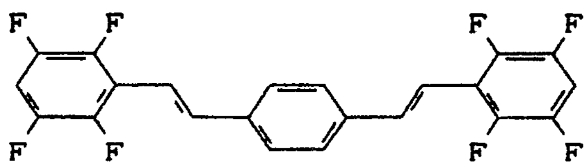 | $\lambda$ = 351,7 nm<br>$\varepsilon$ = 55 800 |
| 14 | 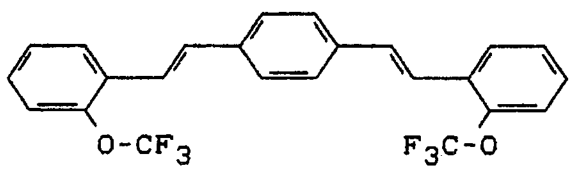 | $\lambda$ = 354,6 nm<br>$\varepsilon$ = 57 000 |
| 15 | 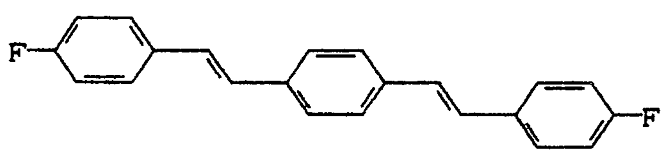 | $\lambda$ = 354,5 nm<br>$\varepsilon$ = 62 000 |
| 16 | 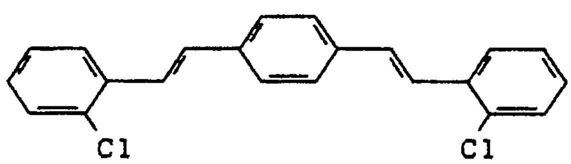 | $\lambda$ = 355,9 nm<br>$\varepsilon$ = 55 000 |

<u>Tabelle</u> (Fortsetzung)

| Beispiel | Formel | Extinktion in DMF |
| --- | --- | --- |
| 17 | 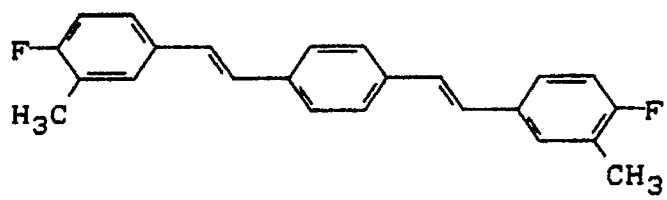 | λ = 355,9 nm<br>ε = 63 500 |
| 18 | 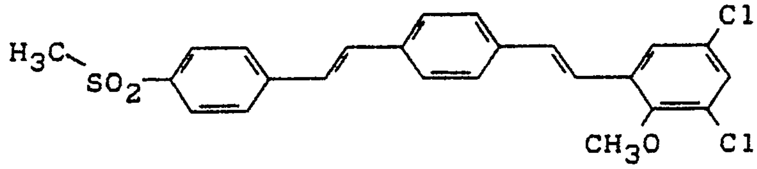 | λ = 367,6 nm<br>ε = 69 000 |
| 19 | 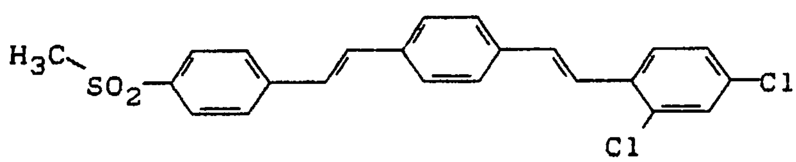 | λ = 367 nm<br>ε = 66 800 |
| 20 | 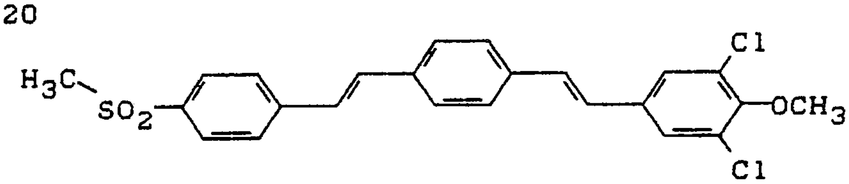 | λ = 367 nm<br>ε = 66 800 |
| 21 | 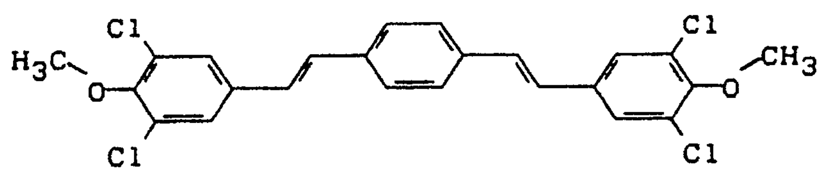 | λ = 363,6 nm<br>ε = 73 700 |

<u>Tabelle</u> (Fortsetzung)

| Beispiel | Formel | Extinktion in DMF |
|---|---|---|
| 22 | 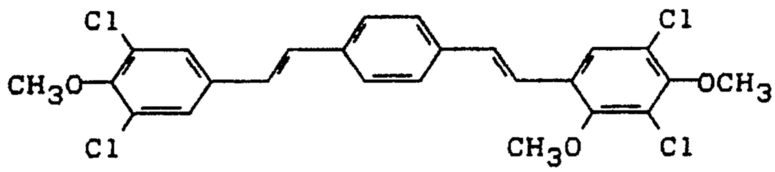 | λ = 365 nm<br>ε = 69 600 |
| 23 | 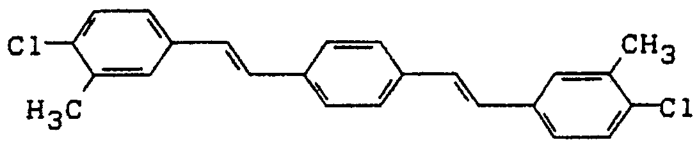 | λ = 363 nm<br>ε = 73 000 |
| 24 | 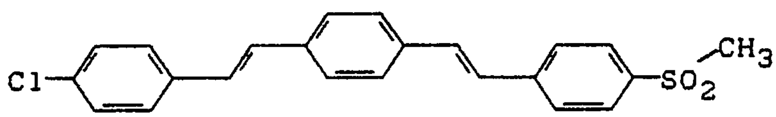 | λ = 362,9 nm<br>ε = 83 700 |
| 25 | 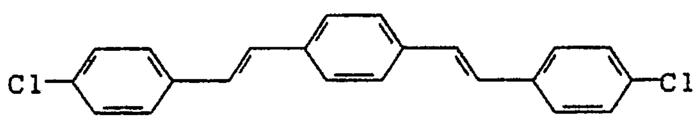 | λ = 362,3 nm<br>ε = 71 500 |
| 26 | 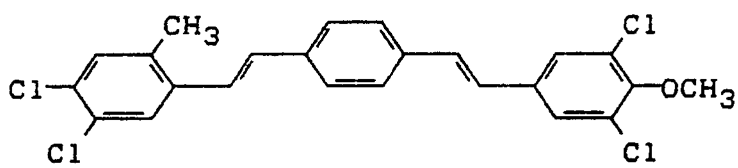 | λ = 361,7 nm<br>ε = 55 000 |
| 27 | 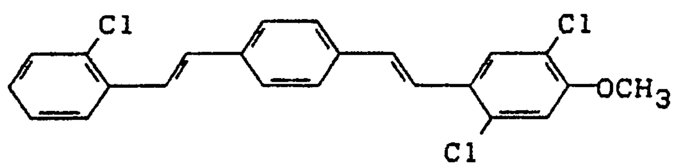 | λ = 361,7 nm<br>ε = 60 000 |

<u>Tabelle</u> (Fortsetzung)

| Beispiel | Formel | Extinktion in DMF |
|---|---|---|
| 28 | 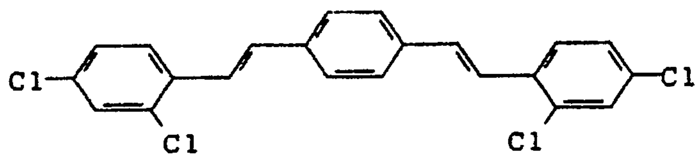 | λ = 361,7 nm<br>ε = 60 000 |
| 29 | 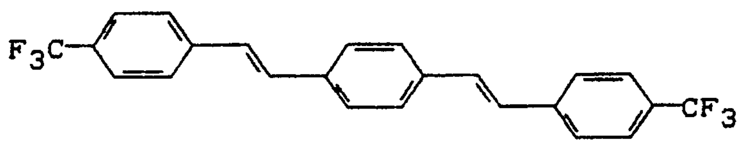 | λ = 361 nm<br>ε = 69 600 |
| 30 | 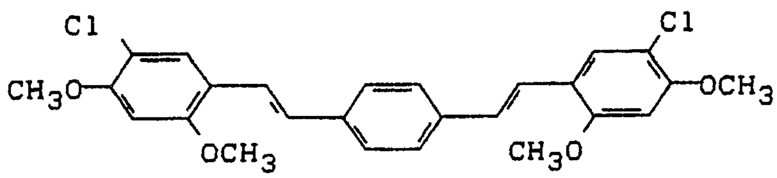 | λ = 582,4 nm<br>ε = 61 500 |
| 31 | 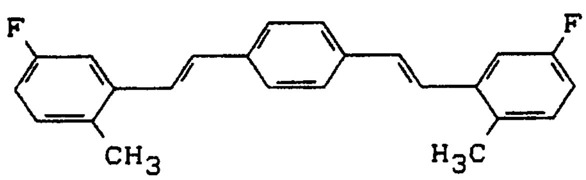 | λ = 358,4 nm<br>ε = 61 000 |
| 32 | 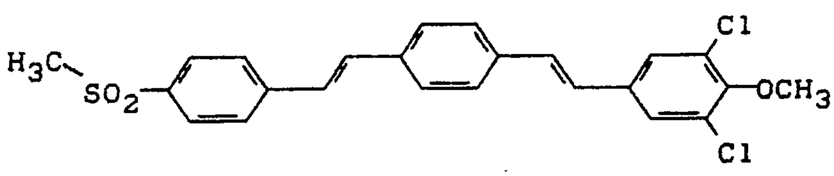 | λ = 367 nm<br>ε = 80 000 |

<u>Tabelle</u> (Fortsetzung)

| Beispiel | Formel | Extinktion in DMF |
|---|---|---|
| 33 | 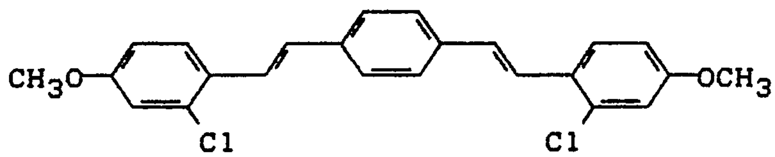 | λ = 367,6 nm<br>ε = 71 000 |
| 34 | 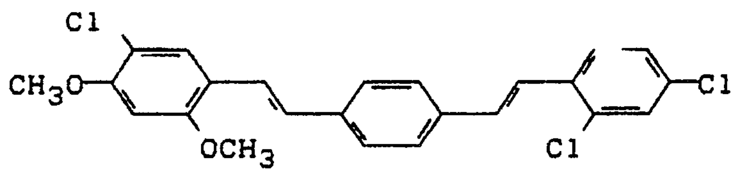 | λ = 366,3 nm<br>ε = 58 500 |
| 35 | 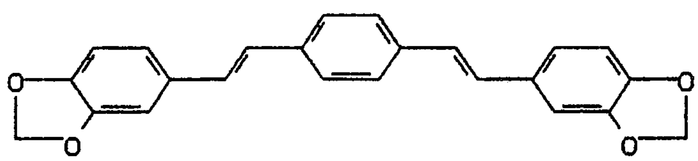 | λ = 373,4 nm<br>ε = 68 500 |
| 36 | 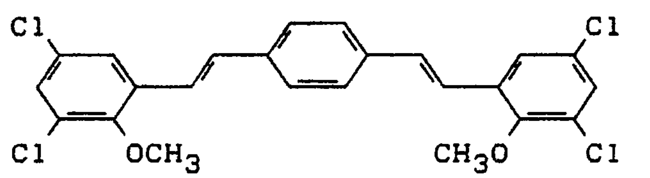 | λ = 366,3 nm<br>ε = 54 500 |
| 37 | 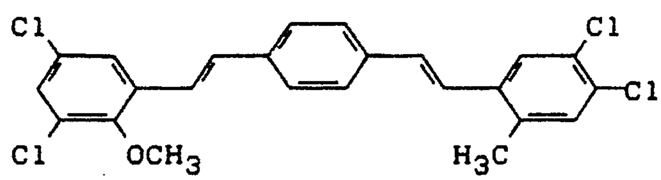 | λ = 365,3 nm<br>ε = 58 000 |

Tabelle (Fortsetzung)

| Beispiel | Formel | Extinktion in DMF |
|---|---|---|
| 38 | 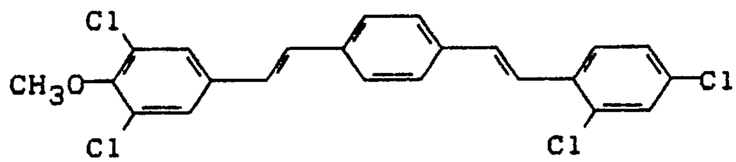 | λ = 363,11nm<br>ε = 71 000 |
| 39 | 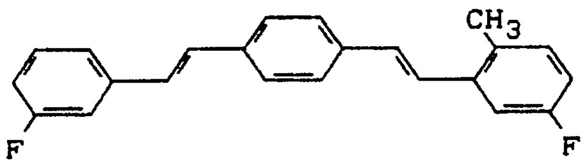 | λ = 355,9 nm<br>ε = 61 200 |
| 40 | 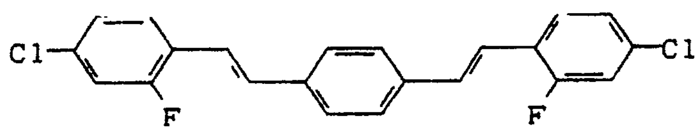 | λ = 364,3 nm<br>ε = 62 500 |
| 41 | 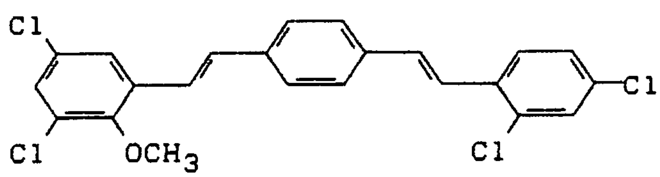 | λ = 363,1 nm<br>ε = 67 500 |
| 42 | 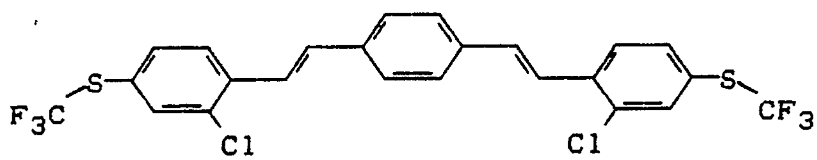 | λ = 370,4 nm<br>ε = 58 000 |
| 43 | 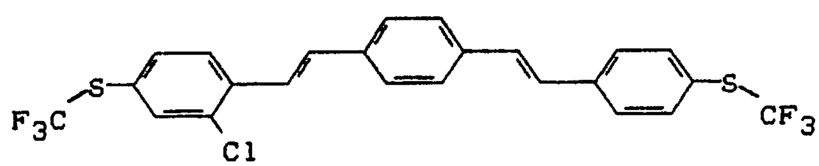 | λ = 368,3 nm<br>ε = 60 000 |

Tabelle (Fortsetzung)

| Beispiel | Formel | Extinktion in DMF |
|---|---|---|
| 44 | 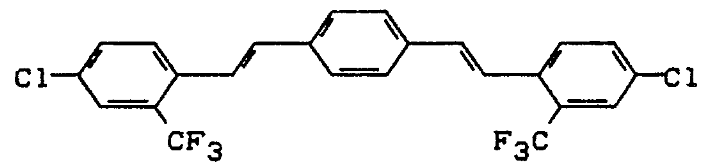 | λ = 347,8 nm<br>ε = 52 000 |
| 45 | 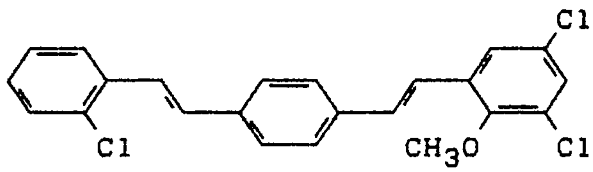 | λ = 358,4 nm<br>ε = 62 500 |
| 46 | 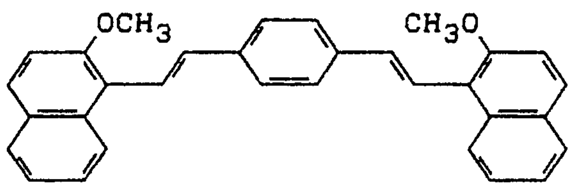 | λ = 381,7 nm<br>ε = 42 000 |
| 47 | 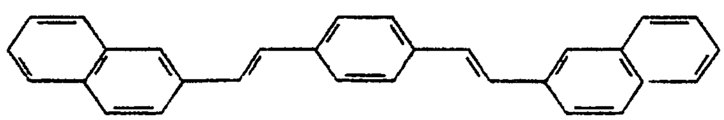 | λ = 375,2 nm<br>ε = 66 000 |
| 48 | 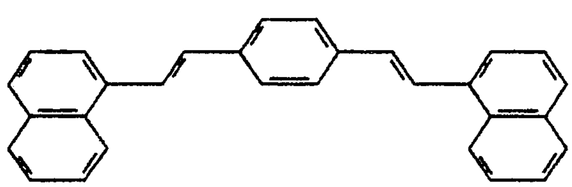 | λ = 373,8 nm<br>ε = 52 000 |

Beispiel 49

Durch Zusammenrühren von
60 Teilen China Clay SPS
40 Teilen Calciumcarbonat
20 Teilen einer anionischen Kunststoffdispersion eines acrylsäureesterhaltigen Mischpolymerisats mit einem Feststoffgehalt von ca. 50 % (z.B. Acronal S 320 D® der BASF)
5 Teilen Polyacrylsäureester als Cc-Binder (40 %ig) (z.B. Acrosol 40 D® der BASF)
80 Teilen Wasser
wird eine Papierstreichmasse mit einem Feststoffgehalt von ca. 55 % hergestellt, deren pH-Wert mit Natronlauge auf 9 eingestellt wird.

Zu 1 kg dieser Streichmasse werden 10 g, 20 g, 40 g, 60 g bzw. 100 g einer 25 %igen Dispersion des optischen Papieraufhellers des Beispiels 1b) eingerührt.

Nach dem Aufstreichen der aufgestellten Streichmasse auf Papier mit Hilfe einer Handrakel oder einer Versuchsstreichanlage und nach Trocknen bei 80 °C erhält man gestrichene Papiere, die je nach Aufhellerzusatz einen deutlichen bis hervorragenden Aufhellungseffekt zeigen. Im Gegensatz zu üblichen Papieraufhellern ist auch bei höheren Aufhellerzusätzen wie z.B. 50 g/kg Streichfarbe ohne Co-Binder noch eine deutliche Steigerung des Weißgrades erzielbar.

Tabelle 2 zeigt den CIE-Weißgrad der gestrichenen Papiere.

Tabelle 2

| 25 %ige Aufhellerdispersion (Beispiel 1b) (g/kg Streichfarbe) | CIE-Weißgrad |
|---|---|
| - | 63,0 |
| 10 | 88,0 |
| 20 | 99,0 |
| 40 | 113,2 |
| 60 | 121,2 |
| 100 | 128,3 |

## Ansprüche

1. Weißtönerhaltige Papierstreichmassen enthaltend als Weißtöner Verbindungen der Formel

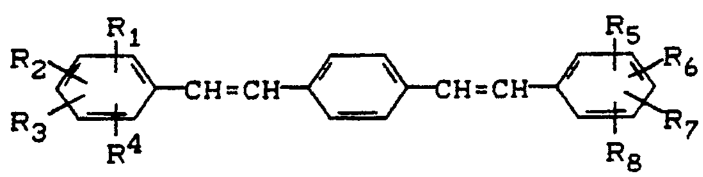

(I)

worin - unabhängig voneinander - $R_1$, $R_2$, $R_3$ und $R_5$, $R_6$, $R_7$ H, F, Cl oder $CH_3$ und $R_4$ und $R_8$ H, O-$C_1$-$C_4$-Alkyl, S-$C_1$-$C_4$-Alkyl, $SO_2C_1$-$C_4$-Alkyl, $CF_3$, $OCF_3$ oder $SCF_3$ bedeuten,
wobei zwei benachbarte Reste auch für $-OCH_2O-$ oder -CH=CH-CH=CH- stehen können.

2. Papierstreichmassen gemäß Anspruch 1, dadurch gekennzeichnet, daß sie die Verbindung der Formel

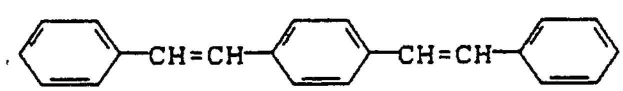

enthalten.

3. Papierstreichmassen gemäß Anspruch 1, dadurch gekennzeichnet, das sie die Verbindung der Formel

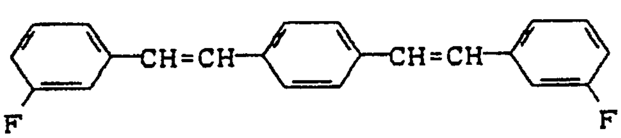

enthalten.

4. Papierstreichmassen gemäß Anspruch 1, dadurch gekennzeichnet, das die durchschnittlichen Teilchengröße der Weißtöner kleiner 5 µm, vorzugsweise kleiner 0,5 µm beträgt.

5. Verbindungen der Formel

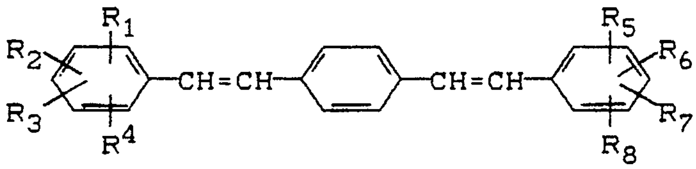

( I )

worin

$R_4$ und $R_8$ F, $CF_3$, $OCF_3$ oder $SCF_3$ bedeutet und die übrigen Reste die oben genannte Bedeutung haben, mit der Maßgabe, daß diese Reste nicht für Wasserstoff stehen, wenn $R_4$/$R_8$ F oder $CF_3$ bedeuten.

6. Verbindung der Formel

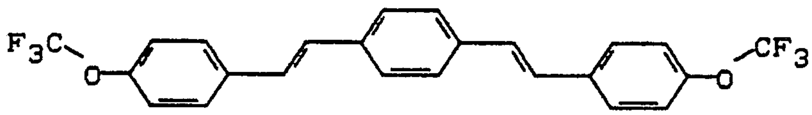

7. Verbindung der Formel

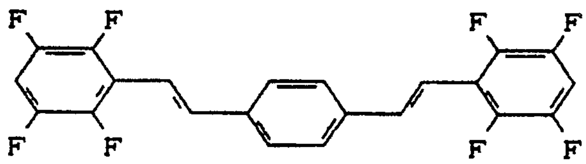

Europäisches Patentamt

# EUROPÄISCHER RECHERCHENBERICHT

Nummer der Anmeldung

**EP 90 12 0276**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CH-A-4 113 29 (BASF)<br>* Anspruch 1; Beispiel 9 * | 1 | D 21 H 21/30<br>C 07 C 43/225<br>C 07 C 25/24<br>C 07 C 15/58<br>C 07<br>C 317/22<br>C 07 C 317/14<br>C 07 C 323/09 |
| X | FR-A-2 214 686 (CIBA-GEIGY)<br>* Anspruch 1; Seite 13, Zeilen 13-18 * | 1 | |
| X | US-A-4 158 099 (A.E. SIEGRIST et al.)<br>* Spalte 23, Zeilen 55-63; Beispiel 5; Spalte 32, Zeile 41 - Spalte 35, Zeile 45 * | 1 | |
| A | DE-A-2 535 102 (NIPPON KAYAKU)<br>* Ansprüche 1-15 * | 1-7 | |
| A | EP-A-0 144 640 (BAYER)<br>* Ansprüche 1-8 * | 1-7 | |
| A | EP-A-0 065 125 (BAYER)<br>* Ansprüche 1-3 * | 1-7 | |
| A | EP-A-0 302 441 (BASF)<br>* Ansprüche 1-3 * | 1-7 | **RECHERCHIERTE SACHGEBIETE (Int. Cl.5)**<br>D 21 H 21/00<br>C 07 C 43/00<br>C 07 C 25/00<br>C 07 C 317/00<br>C 07 C 323/00<br>C 07 C 15/00 |

Der vorliegende Recherchenbericht wurde für alle Patentansprüche erstellt

| Recherchenort | Abschlußdatum der Recherche | Prüfer |
|---|---|---|
| Den Haag | 08 Februar 91 | ZERVAS B. |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument